# EUROPÄISCHE PATENTSCHRIFT

(11) **EP 0 559 034 B1**
(45) Veröffentlichungstag und Bekanntmachung des Hinweises auf die Patenterteilung: **14.04.1999**
(21) Anmeldenummer: 93102700.7
(22) Anmeldetag: 20.02.1993
(51) Int. Cl.: C07D 209/48, C07C 271/18, C07D 207/08, C07B 41/06

(54) **Verfahren zur Herstellung von Aldehyden**
Process for the preparation of aldehydes
Procédé de préparation d'aldéhydes

(30) Priorität: 03.03.1992 CH 664/92
(43) Veröffentlichungstag der Anmeldung: 08.09.1993
(73) Patentinhaber: F. HOFFMANN-LA ROCHE AG, 4002 Basel (CH)
(72) Erfinder: Hilpert, Hans, CH-4153 Reinach (CH)
(74) Vertreter: Witte, Hubert, Dr.

(56) Entgegenhaltungen:
- REC. TRAV. CHIM. Bd. 90, Nr. 12 , 1971 , THE HAGUE Seiten 1323 - 1325 J.A. PETERS AND H. VAN BEKKUM
- SYNTHESIS Nr. 11 , November 1976 Seiten 767 - 768 ALBERT W. BURGSTAHLER ET. AL.
- FIESER L. UND FIESER M.: "Reagents for Organic Synthesis" , , JOHN WILEY & SONS, INC., USA, 1967, S.355
- MARCH J.: "Advanced Organic Chemistry" , , JOHN WILEY & SONS, INC., 3RD EDITION, 1985, S.346
- FIESER L. UND FIESER M.: "Reagents for Organic Synthesis" , , JOHN WILEY & SONS, INC., USA, 1967, S.355
- MARCH J.: "Advanced Organic Chemistry" , , JOHN WILEY & SONS, INC., 3RD EDITION, 1985, S.346

## Beschreibung

Die Herstellung von Aldehyden durch katalytische Hydrierung von Carbonsäurehalogeniden, insbesondere -chloriden, ist als Rosenmund-Reaktion bekannt. Bei dieser Reduktion entsteht Halogen-(Chlor-)wasserstoff, der die Anwendung der Reaktion problematisch gestaltet. Einerseits kann nämlich der entstehende Halogenwasserstoff die Ausbeute an dem gewünschten Aldehyd, sofern dieser säurelabil ist, vermindern oder die Reaktion für die Herstellung solcher Aldehyde gänzlich ungeeignet machen. Andererseits können bei der Entfernung des Halogenwasserstoffs aus dem Reaktionsgemisch, z.B. im Wasserstoffstrom, aus Sicherheitsgründen und in Anbetracht der korrosiven Eigenschaften des Halogenwasserstoffs besondere technische Massnahmen erforderlich werden.

Die Neutralisation des Halogenwasserstoffs durch Zusatz üblicherweise verwendeter Basen bewirkt schlechte Ausbeuten, wenn Carbonsäurehalogenide eingesetzt werden müssen, die basen-labil sind.

Der Artikel von J. A. Peters et al. "A Note on the Rosenmund Reduction of Acid Chlorides" in Recueil Trav. Chim. , 90(12), (1971), S. 1323-1325 beschreibt die katalytische Hydrierung von Carbonsäurechloriden zu Aldehyden unter Einsatz von Ethyldiisopropylamin als H⁺-Fänger.

Der Artikel von A. W. Burgstahler et al. "Improved Modification of the Rosenmund Reduction" in Synthesis, 11, November 1976, S. 767 und 768, beschreibt die katalytische Hydrierung von Carbonsäurechloriden zu Aldehyden unter Einsatz von 2,6-Dimethylpyridin als H⁺-Fänger.

Beide der oben genannten Artikel erwähnen jedoch nicht den Einsatz von Alkylenoxid als H⁺-Fänger.

Alkylenoxide sind als nichtalkalische Fänger für Halogenwasserstoffe bereits bekannt z. B. aus dem Lehrbuch für organische Chemie Fieser L. und Fieser M:, "Reagents for Organic Synthesis", 1967, John Wiley & Sons, Inc., USA, S. 355.. Bei der Umsetzung von Halogenwasserstoff mit Alkylenoxid entsteht Halogenalkohol.

Es wurde nun gefunden, dass die katalytische Hydrierung von Carbonsäurechloriden zur Herstellung von Aldehyden vorteilhaft in Gegenwart von Alkylenoxiden durchgeführt werden kann.

Das erfindungsgemässe Verfahren ist besonders geeignet für die Herstellung säure- oder basenlabiler Aldehyde, wie α-Aminoaldehyde. Weitere Beispiele säurelabiler Aldehyde sind Aldehyde, die säurelabile Gruppen wie Carbamatgruppen, z.B. 2-Trimethylsilyläthylcarbamat, tert.-Butylcarbamat- und 1-Methyl-1-(4-biphenylyl)äthylcarbamatgruppen; oder Aminoacetalgruppen, wie N-Methoxymethylamino, Pivaloyloxymethylamino oder N-Tetrahydropyranylamino; oder Phosphinamidgruppen wie N-Diphenylphosphinylamino enthalten.

Die Erfindung betrifft somit ein Verfahren zur Herstellung von Aldehyden durch katalytische Reduktion von Carbonsäurechloriden mit Wasserstoff in Gegenwart eines Halogenwasserstoff-Fängers und ist dadurch gekennzeichnet, dass als Halogenwasserstoff-Fängers ein Alkylenoxid eingesetzt wird.

Bevorzugt führt man die Reduktion in Gegenwart von C₂₋₆-Alkylenoxiden, insbesondere Butylen-, Propylen- oder Aethylenoxid durch.

Das erfindungsgemässe Verfahren wird zweckmässig in Gegenwart eines inerten, organischen Lösungsmittels durchgeführt. Beispiele solcher Lösungsmittel sind Kohlenwasserstoffe, wie Petroläther oder Toluol; oder halogenierte Kohlenwasserstoffe, wie Methylenchlorid. Im übrigen kann das erfindungsgemässe Verfahren unter den für die Rosenmund-Reaktion an sich bekannten Reaktionsbedingungen durchgeführt werden. Vorzugsweise arbeitet man bei Raumtemperatur und Normaldruck. Als Katalysator können übliche Edelmetallkatalysatoren, insbesondere Palladium, verwendet werden, zweckmässig auf Trägern, wie BaSO₄ oder Kohle. Bevorzugt ist die Verwendung von Palladium auf Kohle, z.B. 5% Pd auf Kohle. Aus dem nach der Hydrierung erhaltenen Reaktionsgemisch kann der gebildete Aldehyd in an sich bekannter Weise, z.B. durch Extraktion, abgetrennt werden.

Die Erfindung wird durch die nachstehenden Beispiele weiter erläutert.

### Beispiel 1

Zu einer Lösung von 0,5 Mol 3-Phenyl-2(S)-phthalimidopropionyl chlorid in 1200 ml Toluol wurden 1 Mol 1,2-Butylenoxid und 23,5 g Pd/C (5% Pd) gegeben. Die Suspension wurde unter kräftigem Rühren 17 Stunden bei Raumtemperatur und Atmosphärendruck hydriert, wobei 11,3 l Wasserstoff aufgenommen wurden. Danach wurde die Suspension über ein Filterhilfsmittel filtriert und der Rückstand mit Toluol gewaschen. Filtrat und Waschflüssigkeit wurden vereinigt und unter Rühren mit einer Lösung von 0,5 Mol Natriumpyrosulfit in 1 l Wasser versetzt. Nach 4,5 Stunden Rühren bei Raumtemperatur wurden die Phasen getrennt. Die wässrige Phase wurde mit 500 ml Toluol gewaschen. Die Toluolphasen wurden mit 350 ml Wasser gewaschen. Die vereinigten wässrigen Phasen wurden mit 1400 ml Toluol und 420 ml 3N Schwefelsäure versetzt und 6 Stunden bei 60° gerührt. Danach wurden die Phasen getrennt und die wässrige Phase mit 500 ml Toluol extrahiert. Die Toluolphasen wurden mit Wasser gewaschen, vereinigt, über MgSO₄ getrocknet und eingedampft. Man erhielt 97,8 g (70%) (S)-α-Benzyl-1,3-dioxo-2-isoindolinacetaldehyd als weisser Feststoff, Schmelzpunkt 115-117°, [α]_{D};²⁰-200° (1% in Aethylacetat).

### Beispiel 2

In Analogie zu Beispiel 1 kann aus 2-tert.-Butoxycarbonylamino-3-cyclohexylpropionylchlorid der 3-Cyclohexyl-2-(tert.-butoxycarbonylamino)-propionaldehyd erhalten werden.

### Beispiel 3

In Analogie zu Beispiel 1 kann aus N-Phthaloyl-leucylchlorid das N-Phthaloyl-leucinal erhalten werden.

### Beispiel 4

In Analogie zu Beispiel 1 kann aus 1-tert.-Butoxycarbonyl-pyrrolidin-2-carbonylchlorid der 1-tert.-Butoxycarbonyl-pyrrolidin-2-carbaldehyd erhalten werden.

## Patentansprüche

1. Verfahren zur Herstellung von Aldehyden durch katalytische Reduktion von Carbonsäurehalogeniden mit Wasserstoff, dadurch gekennzeichnet, dass die Reduktion in Gegenwart eines Alkylenoxids ausgeführt wird.

2. Verfahren nach Anspruch 1, wobei das Alkylenoxid Butylenoxid, Propylenoxid oder Aethylenoxid ist.

3. Verfahren nach Anspruch 1 oder 2 zur Herstellung säure- oder basen-labiler Aldehyde, insbesondere von alpha-Aminoaldehyden.

## Claims

1. A process for the manufacture of aldehydes by the catalytic reduction of a carboxylic acid halide with hydrogen, characterized in that the reduction is carried out in the presence of an alkylene oxide.

2. A process according to claim 1, wherein the alkylene oxide is butylene oxide, propylene oxide or ethylene oxide.

3. A process according to claim 1 or 2 for the manufacture of acid-labile or base-labile aldehydes, especially of alpha-aminoaldehydes.

## Revendications

1. Procédé de préparation d'aldéhydes par la réduction catalytique d'halogénures d'acides carboxyliques avec de l'hydrogène, caractérisé en ce que la réduction est effectuée en présence d'un oxyde d'alkylène.

2. Procédé selon la revendication 1, où l'oxyde d'alkylène est l'oxyde de butylène, l'oxyde de propylène ou l'oxyde d'éthylène.

3. Procédé selon la revendication 1 ou la revendication 2 pour préparer des aldéhydes qui sont labiles avec des acides ou des bases, en particulier des alpha-aminoaldéhydes.
